(19) Europäisches Patentamt

European Patent Office

Office européen des brevets

(11) Publication number: **0 485 212 B1**

(12) **EUROPEAN PATENT SPECIFICATION**

(45) Date of publication of patent specification: **19.07.95**

(51) Int. Cl.6: **A61K 7/08**, A61K 7/50, C11D 1/94, C11D 1/83

(21) Application number: **91310301.6**

(22) Date of filing: **07.11.91**

The file contains technical information submitted after the application was filed and not included in this specification

(54) **Detergent composition.**

(30) Priority: **07.11.90 GB 9024162**

(43) Date of publication of application:
**13.05.92 Bulletin 92/20**

(45) Publication of the grant of the patent:
**19.07.95 Bulletin 95/29**

(84) Designated Contracting States:
**AT BE CH DE DK ES FR GB GR IT LI NL SE**

(56) References cited:
EP-A- 0 086 878          EP-A- 0 155 737
EP-A- 0 194 097          EP-A- 0 422 862
WO-A-90/13283           DE-A- 3 724 460
FR-A- 2 214 746          FR-A- 2 594 692
US-A- 4 371 548          US-A- 4 529 605

(73) Proprietor: **UNILEVER PLC**
**Unilever House**
**Blackfriars**
**P.O. Box 68**
**London EC4P 4BO (GB)**

(84) Designated Contracting States:
**GB**

(73) Proprietor: **UNILEVER N.V.**

**Weena 455**
**NL-3013 AL Rotterdam (NL)**

(84) Designated Contracting States:
**BE CH DE DK ES FR GR IT LI NL SE AT**

(72) Inventor: **Edwards, Robert James**
**Rheingoldstrasse 179**
**W-6800 Mannheim (DE)**
Inventor: **Lee, Robert Stanley**
**37 Poulton Road**
**Spital,**
**Wirral L63 9LD (GB)**
Inventor: **Salmon, Tom Matthew Forrest**
**48 Walpole Street**
**Chester CH1 4HG (GB)**

(74) Representative: **Bryant, Tracey**
**Unilever PLC**
**Patent Division**
**Colworth House**
**Sharnbrook**
**Bedford MK44 1LO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with liquid detergent compositions suitable for personal washing. Examples of such compositions are shower gels and bath foams. A requirement for such compositions is that they should generate a lather when used.

Emollient materials are typically used in a skin cream to help restore and maintain the water content of the skin. At least some emollient materials are inherently antifoaming in nature and therefore appear unsuitable for incorporation in a product which is required to generate a lather in use.

EP-A-155737 discloses shampoo compositions intended to have low irritancy. These compositions contain a betaine and primary alkyl sulphate. Some examples contain sodium lauryl sarcosinate and in at least one of these examples the composition also includes emollient material.

EP-A-194097 discloses a skin-cleansing mousse-forming emulsion containing a volatile propellant and intended to be dispensed from a pressurised dispenser. Preferred compositions are said to contain glycerine with alkyl glyceryl ether sulphonate as surfactant plus a co-surfactant which is a sarcosinate, betaine-type surfactant or mixture thereof.

US-A-4371548 is concerned with bath liquids or shower gels and comments on the difficulty of achieving foaming simultaneously with ability to deposit an oil on the skin. The document goes on to teach compositions containing alkyl ether sulphate, possibly accompanied by primary alkyl sulphate and also containing a cosmetically acceptable oil.

GB-A-1438081 corresponding to FR-A-2214746 is concerned with liquid bath oil compositions and comments on the difficulty of achieving copious foam formation simultaneously with emollient qualities. The document goes on to teach compositions containing anionic detergent together with water insoluble emollient oil.

We have now found that it is possible to incorporate emollient materials into a liquid product containing certain detergent active materials.

Thus the present invention provides a detergent composition in the form of an aqueous liquid of viscosity in the range from 10,000 to 1,000 mPas at 10 sec$^{-1}$ rate, comprising:

A - foaming detergent active which is a mixture of:

A1 - at least 5% by weight of the composition of a non-soap anionic detergent which is selected from alkyl ether sulphates, acyl isethionates, alkyl glycerol ether sulphonates, acyl glutamates, acyl peptides, sarcosinates, ester carboxylic acids, $\alpha$-olefin sulphonates, sulphosuccinates, alkyl benzene sulphonates, amides of N-methyl taurine and $\alpha$-sulpho fatty acids;

A2 - at least 1% by weight of the composition of amphoteric detergent selected from betaines, sulphobetaines and amidobetaines; and

B - a skin emollient material.

### A - Detergent Active Materials

As apparent from the above, the detergent active materials employed in the present invention are a mixture of:

A1 - non-soap anionic detergent, and

A2 - amphoteric detergent.

The specified anionic detergent which is required to provide at least 5% by weight of the composition will generally include an alkyl or acyl group of 8 to 20 carbon atoms, together with an anionic group.

The second detergent (A2) is an amphoteric surfactant which may be a betaine, a sulphobetaine or an amido betaine. All of these are zwitterionic.

### B - Skin Emollient Agents

These materials may be chosen from three categories which are

B1 - Skin moisturisers

B2 - Occlusive agents

B3 - Humectants

Skin moisturisers (B1) are compounds which enhance moisture retention by the skin and may also attract water into the skin. Typical moisturising materials include urea, lactic acid, pyrrolidone carboxylic acid, amino acids and salts of the acids mentioned.

The occlusive agents (B2) are substances which form on the skin thin films of limited permeability, serving to hold water within the skin and prevent dehydration. The range of occlusive agents is consider-

EP 0 485 212 B1

able. They are generally hydrophobic oils and waxes. Examples of classes of such agents and individual examples of such agents are:

1. Hydrocarbon oils and waxes. Examples thereof are mineral oil, petrolatum, paraffin, ceresin, ozokerite, microcrystalline wax.

2. Silicone oils, such as dimethyl polysiloxanes, methylphenyl polysiloxanes, silicone glycol copolymers.

3. Triglyceride esters, for example vegetable and animal fats and oils.

4. Glyceride esters and esters such as acetylated monoglycerides, and ethoxylated monoglycerides.

5. Alkyl and alkenyl esters of fatty acids having 10 to 20 carbon atoms. Examples include hexyl laurate, isohexyl laurate, isohexyl palmitate, isopropyl myristate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, cetyl lactate, oleyl myristate, oleyl stearate and oleyl oleate.

6. Fatty alcohols having 10 to 20 carbon atoms. Lauryl, myristyl, cetyl, hexadecyl, stearyl, isostearyl, hydroxystearyl, oleyl, ricinoleyl, behenyl, erucyl, and 2-octyl dodecanyl alcohols are examples of satisfactory fatty alcohols.

7. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols and lanolin alcohols linoleate are illustrative emollients derived from lanolin.

8. Natural waxes, esters thereof and ethoxylated natural waxes, beeswax, spermaceti, myristyl myristate, stearyl stearate, polyoxyethylene sorbitol beeswax, carnauba wax and candelilla wax.

Especially preferred are $C_2$-$C_4$ alkyl esters of $C_{12}$-$C_{18}$ fatty acids, such as isopropyl myristate, and isopropyl palmitate.

Humectants (B3) are especially C2-C6 polyols notably glycerol, sorbitol, propylene glycol and 1,3-butylene glycol. A further example of humectant is polyetheylene glycols having molecular weights of from about 100 to about 1500. Humectants do not themselves form occlusive films but may cooperate with other materials to form a film having occlusive properties. It is therefore desirable that humectants are not the sole category of skin emollient agent present.

The overall amount of emollient agents included in a composition of this invention is preferably in a range from 0.1 to 10% of the composition more preferably 0.2 to 5% by weight of the composition. Somewhat more specifically, it is preferred that the skin emollient agents include occlusive agent (B2) in an amount from 0.1 to 2% by weight of the composition, better 0.2 to 1%. If skin moisturiser (B1) is present the amount is preferably 0.02 to 5%, better 0.05 to 2% of the composition. If humectant (B3) is present the amount will generally not exceed 15% by weight of the composition and it will generally be convenient for humectant to be present in a quantity from 0.1 to 15% better 0.5 to 5 or 8% by weight of the composition.

Particularly preferred weight ratios of the three categories of skin emollient agent are a weight ratio of B1:B2:B3 = 0-15:2-10:5-50

Preferred amounts of the detergent active lie in a range up to 40% by weight of the overall composition, preferably up to 25% by weight. Preferably anionic detergent active (A1) is present in an amount from 5 to 20% by weight of the composition while the second detergent active (A2) is present in an amount from 1 to 15%, preferably 1 to 10% by weight of the composition. The two categories of detergent active A1:A2 are preferably in a weight ratio from 10:1 to 1:10, better 9:1 to 1:2.

The weight ratio of detergent active to skin emollient agents preferably lies in a range from 20:1 to 5:1. Alternatively or additionally the weight ratio of detergent active to occlusive agent lies in a range from 10:1 to 100:1.

Thus, a preferred form of this invention comprises the materials A1, A2 and B1-B3 as explained above in amounts by weight which are:

| A1 | 5-20% |
|----|-------|
| A2 | 1-15% |
| B1 | 0.02-5% |
| B2 | 0.1-2% |
| B3 | 0-15% preferably 0.5 to 8% |

Other materials may be included in compositions according to this invention. In particular, electrolyte or thickening agents or both may be incorporated to control the viscosity of the composition. We prefer that compositions of this invention should have a viscosity in the range from 10,000 to 1000 mPs at 10 sec$^{-1}$

3

shear rate, more preferably 8000 to 2000 mPs.

Thickening agents may be synthetic cross-linked polymers such as cross-linked polyacrylates. Another category of thickening agents are polysaccharides such as vegetable mucins, alginates, cellulose derivatives and xanthan gums. The amount of any thickening agent is likely to range from 0.01 to 5% by weight of the composition and likely narrower limits are 0.05 to 2% by weight of the composition.

Further materials which are likely to be included are pearlescers or opacifiers, perfumes, colorants and antioxidants. Preservatives may well be included.

Free fatty acids typically of 8 to 22 carbon atoms may be present or may be avoided. Soaps are possible but are preferably avoided. If soap or fatty acid is present the amount of each is unlikely to exceed 5% by weight of the composition and more particularly may be in an amount from 0.5 to 2.5% by weight of the composition.

One feature which is possible within this invention is that the skin emollient agents (B) may be capable of incorporation at the same relative proportions into a stable emulsion. The concentration of these agents in such an emulsion would be the same or more probably greater than their concentration in the detergent composition of the invention.

This feature creates the possibility of incorporating the formulation of one product form, namely a skin moisturising liquid or cream, into another product form, namely the liquid detergent composition of the invention.

When the invention takes such a form it can be defined as an aqueous liquid detergent composition containing

foaming detergent active and

the non-aqueous constituents of a moisturising emulsion which comprises skin emollient agents.

Such a moisturising emulsion might include other materials which are also incorporated into the detergent composition, for example thickeners, opacifiers or pearlescers.

Forms of the invention which are of special interest are those in which the detergent active comprises $C_8$ to $C_{22}$ fatty acyl isethionate. Fatty acyl isethionate is known as mild to the skin but is poorly soluble. We have observed that combinations of fatty acyl isethionate with an amphoteric surfactant or a polar nonionic surfactant can give solutions which are clear and isotropic (if in the absence of opacifier, pearlescer or other non-detergent insolubles).

Consequently forms of this invention can be defined as detergent compositions containing

    A) 3 to 40% of detergent composed of

        A1) at least 2% of $C_8$-$C_{22}$ acyl isethionate

        A2) at least 1% of amphoteric detergent selected from betaines, sulphobetaines and amidobetaines;

    B) 0.1 to 10% of skin emollient agents

wherein the ratio of acyl isethionate to detergent A2 is from 10:1 to 1:4, better 5:1 to 1:2, and the detergent actives present in the composition form a clear solution in distilled water at the same concentrations.

The detergent actives present may include additional anionic detergent (A1) and the amount may be such as to keep the ratio of total anionic (A1) to second detergent (A2) within a range of 10:1 to 1:2.

Method of Preparation

The compositions of this is invention can be prepared by simply mixing their constituents with water. Premixes of some components may be made and combined, if convenient. It will generally be desirable to arrange the order of addition so that thickening occurs after most of the mixing has taken place.

Example 1

Shower gels were prepared with formulations as set out in Table 1 below.

In each case the anionic detergent (A1) was sodium lauryl ether sulphate (SLES) with an average of three ethylene oxide groups per molecule. The second detergent (A2) was coconut amido propyl betaine (CAPB) opacifier was a polystyrene latex. The alternative to this was a pearlescent agent which was ethylene glycol distearate (EGDS).

"NMF" denotes a mixture of materials sold as equivalent to a natural moisturising factor in skin. Such a mixture is available commercially as HYDROVITON (trade mark) from Dragoco, LACTIL (trade mark) from Goldsmith or PRODEW (trade mark) from Ajinomoto. The NMF used contained 40% of free amino acids, 12% pyrrolidone carboxylic acid, 7% urea and 12% lactate, the balance being made up by other constituents. Thus this material contained at least 70% of skin moisturising agents.

TABLE 1

| Formulation: | A | B | C | D |
|---|---|---|---|---|
| Ingredients | | % by weight | | |
| SLES | 12 | 12 | 12 | 12 |
| CAPB | 3 | 3 | 3 | 3 |
| Opacifier | 1 | 1 | - | - |
| Pearlescer | - | - | 4 | 4 |
| Isopropyl palmitate | - | 0.5 | - | 0.5 |
| Glycerol | - | 1.25 | - | 1.25 |
| NMF | - | 0.25 | - | 0.25 |
| Preservative | 0.07 | 0.07 | 0.07 | 0.07 |
| Sodium chloride | 4.0 | 4.0 | 4.0 | 4.0 |
| Water | - - balance to 100% -- | | | |

The four formulations were assessed by a panel of ten persons. Each panelist wore surgical gloves. The gloves were first washed with a standard detergent composition in running water at 30°C. One gram of the test composition was then dispensed from a syringe into the palm of each panelist. The panelists were asked to wash their hands as they would normally, using water of 26° French hardness (FH) at 30°C. The panelists were asked to score the lather volume and perceived creaminess of the lather. The actual amount of lather produced was assessed by collecting the lather in an inverted funnel which is then used to transfer the lather to a graduated cylinder.

The ability of the compositions to generate foam was also determined by the following test. 5 grams of the formulation was put into a graduated separating funnel. 50 grams of water is then added. This water was of 26° FH at 25°C. The funnel is shaken for 10 seconds and the foam level measured and noted as "initial foam". The solution below the foam is drained off, a further 50 grams of water is added, the funnel is shaken again and foam volume noted. The draining off of solution, addition of water and shaking is repeated until all foam has disappeared. The number of additions of 50 gram aliquots of water is referred to as "number of rinses" and is an indication of the amount of water required to rinse the foam. Each time an aliquot of water was added and the separating funnel shaken the amount of foam present was noted. The total of these amounts of foam is referred to as the cumulative foam volume.

Table 2 sets out total of normalised panel scores, cumulative foam volume and number of rinses (i.e. number of aliquots added until no foam remains) for each of the four formulations.

The results show that the inclusion of emollient agents in formulations B and D had little or no adverse effect on foaming. In the case of compositions A and B, the panel test and funnel test give opposite results but it can be seen that the effect of including the emollient agents is much less than the change from opacifier to pearlescer.

TABLE 2

| Formulation: | A | B | C | D |
|---|---|---|---|---|
| Total Normalised Panel Scores: | | | | |
| Lather Volume | 142.7 | 102.3 | 98.1 | 102.1 |
| Lather Creaminess | 111.7 | 105.4 | 103.9 | 99.2 |
| Funnel Test | | | | |
| Cumulative Foam Volume | 6550 | 6900 | 2025 | 2550 |
| No Rinses to Zero Foam | 15 | 13 | 11 | 11 |

Example 2

Creams were prepared using emollient agents and pearlescer or opacifier as used in Example 1. Crosslinked polyacrylate was included. The formulations were as follows:

6

| Formulation: | E | F |
|---|---|---|
| Ingredient | wt% | wt% |
| Glycerol | 5 | 5 |
| Isopropyl palmitate | 2 | 2 |
| NMF | 1 | 1 |
| Pearlescer | 16 | - |
| Opacifier | - | 4 |
| Perfume | 1 | 1 |
| Polyacrylate | 1 | 1 |
| Water | -- balance to 100% -- | |

The method of producing the creams was to mix all of the materials with water, incorporating the polyacrylate in the acid form supplied by the manufacturers, and shaken to form an emulsion. the resulting compositions were viscous stable creams.

Example 3

Liquid compositions were prepared generally as in Example 1 but incorporating a sulphosuccinate in place of betaine. One composition incorporated 25% of glycerol. The formulations were:

| Formulation: | G | H |
|---|---|---|
| Ingredient | % by weight | |
| SLES | 10 | 10 |
| Sulphosuccinate | 2 | 2 |
| Tegobetaine | 3 | 3 |
| Glycerol | - | 25 |
| Perfume | 1.5 | 1.5 |
| Sodium chloride | 1.5 | 1.5 |

The compositions were assessed by panel testing as in Example 1 and the results obtained were as follows:

|  | G | H |
|---|---|---|
|  |  | (with glycerol) |
| Panel Scores: |  |  |
| Lather Volume | 158 | 165 |
| Lather Creaminess | 125 | 148 |

Example 4

Example 3 was repeated, but one formulation contained 10% of sodium lactate. The results were as follows:

| Total Panel Scores | without lactate | with lactate |
|---|---|---|
| Lather Volume | 227 | 188 |
| Lather Creaminess | 162 | 174 |

These Examples 3 and 4 show that up to 25% glycerol and up to 10% lactate can be included without serious adverse effect.

Example 5

A shower gel was prepared generally as Example 1D, but containing a different detergent active system, which includes sodium cocoyl isethionate.

The foaming ability of this composition was compared with two compositions similar to Examples 1C and 1D. The three formulations were as set out in Table 4 below. SLES, CAPB, NMF and pearlescer are the same materials as used in Example 1.

The test procedure was as follows. 2 gram of test composition are dissolved in 20mls of distilled water. 20mls of this solution are put into a 100ml stoppered measuring cylinder. The cylinder is shaken 10 times, swapped to the other hand and shaken 10 times again. The height of the lather is measured immediately. The operation is performed twice for each product, and the results are an average of the two measurements.

The data obtained by this method are included in Table 4 below.

TABLE 4

| Formulation: | I | J | K |
|---|---|---|---|
| Ingredients | % by weight | | |
| Sodium cocoyl isethionate | 5 | - | - |
| SLES | 2 | 13 | 13 |
| CAPB | 8 | 2 | 2 |
| Pearlescer | 4 | 4 | 4 |
| Isopropyl palmitate | 0.5 | - | 0.5 |
| Glycerol | 1.25 | - | 1.25 |
| NMF | 0.25 | - | 0.25 |
| Preservative | 0.07 | 0.07 | 0.07 |
| Sodium chloride | 3.5 | 4.0 | 4.0 |
| Water | -- balance to 100% -- | | |
| Foam height (cm) | 14.7 | 12.5 | 15.4 |

## Claims

1. A detergent composition in the form of an aqueous liquid of viscosity in the range from 10,000 to 1,000 mPas at 10 sec$^{-1}$ shear rate, comprising:
   A - foaming detergent active which is a mixture of:
   A1 - at least 5% by weight of the composition of a non-soap anionic detergent which is selected from alkyl ether sulphates, acyl isethionates, alkyl glycerol ether sulphonates, acyl glutamates, acyl peptides, sarcosinates, ester carboxylic acids, $\alpha$-olefin sulphonates, sulphosuccinates, alkyl benzene sulphonates, amides of N-methyl taurine and $\alpha$-sulpho fatty acids;
   A2 - at least 1% by weight of the composition of amphoteric detergent selected from betaines, sulphobetaines and amidobetaines; and
   B - a skin emollient material.

2. A detergent composition according to claim 1, wherein the skin emollient material (B) is selected from:
   B1 - skin moisturisers
   B2 - occlusive agents
   B3 - humectants
   and mixtures thereof.

3. A detergent composition according to claim 2, wherein the skin moisturiser, if present is selected from urea, lactic acid, pyrrolidone carboxylic acid, amino acids, and salts of these acids.

4. A detergent composition according to claim 2, wherein the occlusive agent, if present, is selected from: hydrocarbon oils and waxes; silicone oils; triglyceride esters; glyceride esters, acetylated monoglycerides, alkoxylated monoglycerides; alkyl and alkenyl esters of fatty acids having 10 to 20 carbon atoms; fatty alcohols having 10 to 20 carbon atoms; lanolin and derivatives thereof; natural waxes, esters thereof and alkoxylated natural waxes.

5. A detergent composition according to claim 2, wherein the humectant, if present, is selected from $C_2$-$C_6$ polyols; polyethylene glycols having molecular weights of from about 100 to about 1500.

6. A detergent composition according to any preceding claim, wherein the skin emollient material (B) is present in an amount of from 0.1 to 10% by weight of the composition.

7. A detergent composition according to claim 2, wherein:
the skin moisturiser (B1), if any, is present in an amount of from 0.02 to 5% by weight, of the composition; the occlusive agent (B2), if any, is present in an amount of from 0.1 to 2% by weight of the composition; and the humectant (B3), if any, is present in an amount of up to 15% by weight of the composition.

8. A detergent composition according to claim 7, wherein the weight ratios of the three categories of skin emollient material are B1:B2:B3 = 0-15:2-10:5-50.

9. A detergent composition according to any preceding claim, wherein the detergent active (A) is present in an amount not exceeding 40% by weight of the composition.

10. A detergent composition according to any one of the preceding claims wherein the non-soap anionic detergent (A1) is present in an amount of from 5 to 20% by weight of the composition, and the amphoteric detergent (A2) is present in an amount of from 1 to 15% by weight of the composition.

11. A detergent composition according to claim 10, wherein the weight ratio of the two categories of detergent active is A1:A2 = 10:1 to 1:10.

12. A detergent composition according to any preceding claim, wherein the weight ratio of foaming detergent active (A) to skin emollient material (B) is A:B = 20:1 to 5:1.

13. A detergent composition according to claim 2, wherein the weight ratio of foaming detergent active (A) to occlusive agent (B2) is A:B2 = 10:1 to 100:1.

14. A detergent composition according to any preceding claim, wherein the skin emollient material comprises a humectant (B3).

15. A detergent composition according to any preceding claim, wherein the skin emollient material comprises a humectant (B3) together with a skin moisturiser (B1).

16. A detergent composition according to any preceding claim, wherein the skin emollient material comprises a humectant (B3) together with an occlusive agent (B2).

17. A detergent composition according to any preceding claim, wherein the skin emollient material comprises:
B1 - 0.02-5% of a skin moisturiser,
B2 - 0.1-2% of an occlusive agent; and
B3 - 0-15% of a humectant.

18. A detergent composition according to any preceding claim, further comprising one or more additional materials selected from thickening agents, pearlescers or opacifiers, perfumes, colourants, antioxidants, preservatives, fatty acids and soaps.

19. A detergent composition according to any preceding claim, wherein the anionic detergent active (A1) comprises $C_8$ to $C_{22}$ fatty acyl isethionate.

**20.** A detergent composition in the form of an aqueous liquid of viscosity in the range from 10,000 to 1,000 mPas at 10 sec$^{-1}$ shear rate, containing:

A) 3 to 40% of detergent composed of

A1) at least 2% of $C_8$-$C_{22}$ acyl isethionate

A2) at least 1% of amphoteric detergent selected from betaines, sulphobetaines and amidobetaines;

B) 0.1 to 10% of a skin emollient material, wherein the weight ratio of acyl isethionate (A1) to detergent (A2) is from 10:1 to 1:4 and the detergent actives present in the composition form a clear solution in distilled water at the same concentrations.

**21.** A detergent composition according to claim 20, wherein the detergent actives present include additional anionic detergent.

**22.** A detergent composition according to claim 21, wherein the amount of additional anionic detergent is such as to keep the weight ratio of total anionic detergent to second detergent (A2) within the range 10:1 to 1:2.

**Patentansprüche**

**1.** Reinigungsmittelzusammensetzung in Form einer wäßrigen Flüssigkeit einer Viskosität im Bereich von 10.000 bis 1000 mPa•s bei einer Scherrate von 10 s$^{-1}$, die die folgenden Bestandteile umfaßt:

A - einen schäumenden Reinigungsmittelaktivstoff in Form eines Gemisches:

A1 - eines aus Alkylethersulfaten, Acylisethionaten, Alkylglycerinethersulfonaten, Acylglutamaten, Acylpeptiden, Sarcosinaten, Estercarbonsäuren, $\alpha$-Olefinsulfonaten, Sulfosuccinaten, Alkylbenzolsulfonaten, Amiden von N-Methyltaurin und $\alpha$-Sulfofettsäuren ausgewählten anionischen Nichtseife-Reinigungsmittels in einer Menge von mindestens 5 Gew.-% der Zusammensetzung und

A2 - eines aus Betainen, Sulfobetainen und Amidobetainen ausgewählten amphoteren Reinigungsmittels in einer Menge von mindestens 1 Gew.-% der Zusammensetzung sowie

B - ein Hauterweichungsmittelmaterial.

**2.** Reinigungsmittelzusammensetzung nach Anspruch 1, wobei das Hauterweichungsmittelmaterial (B) aus

B1: Hautfeuchthaltemitteln,

B2: Abdeckungsmitteln,

B3: Benetzungsmitteln und Gemischen hiervon ausgewählt ist.

**3.** Reinigungsmittelzusammensetzung nach Anspruch 2, wobei das gegebenenfalls vorhandene Hautbenetzungsmittel aus Harnstoff, Milchsäure, Pyrrolidoncarbonsäure, Aminosäuren und Salzen dieser Säuren ausgewählt ist.

**4.** Reinigungsmittelzusammensetzung nach Anspruch 2, wobei das gegebenenfalls vorhandene Abdeckungsmittel aus Kohlenwasserstoffölen und -wachsen, Siliconölen, Triglyceridestern, Glyceridestern, acetylierten Monoglyceriden, alkoxylierten Monoglyceriden, Alkyl- und Alkenylestern von Fettsäuren mit 10 bis 20 Kohlenstoffatomen, Fettalkoholen mit 10 bis 20 Kohlenstoffatomen, Lanolin und Derivaten hiervon, natürlichen Wachsen, Estern hiervon und alkoxylierten natürlichen Wachsen ausgewählt ist.

**5.** Reinigungsmittelzusammensetzung nach Anspruch 2, wobei das gegebenenfalls vorhandene Benetzungsmittel aus $C_2$-$C_6$-Polyolen, Polyethylenglycolen mit Molekulargewichten von etwa 100 bis etwa 1500 ausgewählt ist.

**6.** Reinigungsmittelzusammensetzung nach einem der vorhergehenden Ansprüche, wobei das Hauterweichungsmittelmaterial (B) in einer Menge von 0,1 bis 10 Gew.-% der Zusammensetzung vorhanden ist.

**7.** Reinigungsmittelzusammensetzung nach Anspruch 2, wobei das gegebenenfalls vorhandene Hautbenetzungsmittel (B1) in einer Menge von 0,02 bis 5 Gew.-% der Zusammensetzung, das gegebenenfalls vorhandene Abdeckungsmittel (B2) in einer Menge von 0,1 bis 2 Gew.-% der Zusammensetzung und das gegebenenfalls vorhandene Benetzungsmittel (B3) in einer Menge von bis zu 15 Gew.-% der Zusammensetzung vorhanden sind.

**8.** Reinigungsmittelzusammensetzung nach Anspruch 7, wobei das Gew.-Verhältnis der drei Kategorien des Hauterweichungsmittelmaterials B1/B2/B3 0-15/2-10/5-50 beträgt.

**9.** Reinigungsmittelzusammensetzung nach einem der vorhergehenden Ansprüche, wobei der Reinigungsmittelaktivstoff (A) in einer Menge nicht über 40 Gew.-% der Zusammensetzung vorhanden ist.

**10.** Reinigungsmittelzusammensetzung nach einem der vorhergehenden Ansprüche, wobei das anionische Nichtseife-Reinigungsmittel (A1) in einer Menge von 5 bis 20 Gew.-% der Zusammensetzung und das amphotere Reinigungsmittel (A2) in einer Menge von 1 bis 15 Gew.-% der Zusammensetzung vorhanden sind.

**11.** Reinigungsmittelzusammensetzung nach Anspruch 10, wobei das Gew.-Verhältnis der beiden Kategorien des Reinigungsmittelaktivstoffs A1/A2 10/1 bis 1/10 beträgt.

**12.** Reinigungsmittelzusammensetzung nach einem der vorhergehenden Ansprüche, wobei das Gew.-Verhältnis des schäumenden Reinigungsmittelaktivstoffs (A) zu dem Hauterweichungsmittelmaterial (B) A/B = 20/1 bis 5/1 beträgt.

**13.** Reinigungsmittelzusammensetzung nach Anspruch 2, wobei das Gew.-Verhältnis des schäumenden Reinigungsmittelaktivstoffs (A) zu dem Abdeckungsmittel (B2) A/B2 = 10/1 bis 100/1 beträgt.

**14.** Reinigungsmittelzusammensetzung nach einem der vorhergehenden Ansprüche, wobei das Hauterweichungsmittelmaterial ein Benetzungsmittel (B3) umfaßt.

**15.** Reinigungsmittelzusammensetzung nach einem der vorhergehenden Ansprüche, wobei das Hauterweichungsmittelmaterial ein Benetzungsmittel (B3) zusammen mit einem Hautfeuchthaltemittel (B1) umfaßt.

**16.** Reinigungsmittelzusammensetzung nach einem der vorhergehenden Ansprüche, wobei das Hauterweichungsmittelmaterial ein Benetzungsmittel (B3) zusammen mit einem Abdeckungsmittel (B2) umfaßt.

**17.** Reinigungsmittelzusammensetzung nach einem der vorhergehenden Ansprüche, wobei das Hauterweichungsmittelmaterial
B1: 0,02-5 Gew.-% eines Hautfeuchthaltemittels,
B2: 0,1-2 Gew.-% eines Abdeckungsmittels und
B3: 0-15 Gew.-% eines Benetzungsmittels umfaßt.

**18.** Reinigungsmittelzusammensetzung nach einem der vorhergehenden Ansprüche, die des weiteren ein oder mehrere weitere Materialien, die aus Dickungsmitteln, einem Perlmuttglanz verleihenden Mitteln oder Opazifierungsmitteln, Duftstoffen, Färbemitteln, Antioxidantien, Konservierungsmitteln, Fettsäuren und Seifen ausgewählt sind, umfaßt.

**19.** Reinigungsmittelzusammensetzung nach einem der vorhergehenden Ansprüche, wobei der anionische Reinigungsmittelaktivstoff (A1) ein $C_8$-$C_{22}$-Fettacylisethionat umfaßt.

**20.** Reinigungsmittelzusammensetzung in Form einer wäßrigen Flüssigkeit einer Viskosität im Bereich von 10.000 bis 1000 mPa•s bei einer Scherrate von 10 $s^{-1}$, die
A) 3 bis 40% Reinigungsmittel aus
A1) mindestens 2% eines $C_8$-$C_{22}$-Acylisethionats
A2) mindestens 1% eines aus Betainen, Sulfobetainen und Amidobetainen ausgewählten amphoteren Reinigungsmittels, sowie
B) 0,1 bis 10% Hauterweichungsmittelmaterial enthält, wobei das Gew.-Verhältnis Acylisethionat (A1)/Reinigungsmittel (A2) 10/1 bis 1/4 beträgt und die in der Zusammensetzung vorhandenen Reinigungsmittelaktivstoffe in destilliertem Wasser bei denselben Konzentrationen eine klare Lösung bilden.

**21.** Reinigungsmittelzusammensetzung nach Anspruch 20, wobei die vorhandenen Reinigungsmittelaktivstoffe ein weiteres anionisches Reinigungsmittel umfassen.

EP 0 485 212 B1

**22.** Reinigungsmittelzusammensetzung nach, Anspruch 21, wobei die Menge eines zusätzlichen anionischen Reinigungsmittels derart ist, daß das Gew.-Verhältnis gesamtes anionisches Reinigungsmittel/zweites Reinigungsmittel (A2) in einem Bereich von 10/1 bis 1/2 gehalten wird.

**Revendications**

**1.** Composition détergente sous la forme d'un liquide acqueux de viscosité comprise dans l'intervalle de 10 000 à 1 000 mPas à une vitesse de cisaillement de 10 s$^{-1}$, comprenant :

A - un agent détergent actif moussant qui est un mélange de :

A1 - au moins 5 % en poids de la composition d'un détergent anionique non savonneux qui est sélectionné parmi des sulfates éther alkyles, des iséthionates acyles, des sulfonates éther glycérol alkyle, des glutamates acyles, des peptides acyles, des sarcosinates, des acides d'ester carboxylique, des sulfonates d'-oléfine, des sulfosuccinates, des sulfonates de benzène alkyl, des amides de taurine N-méthyl et des $\alpha$-sulfo acides gras ;

A2 - au moins 1 % en poids de la composition d'un détergent amphotère sélectionné parmi les bétaïnes, les sulfobétaïnes et les amidobétaïnes ; et

B - un matériau émollient de la peau.

**2.** Composition détergente selon la revendication 1, dans laquelle le matériau émollient de la peau (B) est sélectionné parmi :

B1 - des agents d'humidification de la peau

B2 - des agents d'occlusion

B3 - des agents d'humectation

et des mélanges de ceux-ci.

**3.** Composition détergente selon la revendication 2, dans laquelle l'agent d'humidification de la peau, si présent, est sélectionné parmi l'urée, l'acide lactique, l'acide carboxylique de pyrrolidone, des acides aminés, et des sels de ces acides.

**4.** Composition détergente selon la revendication 2, dans laquelle l'agent d'occlusion, si présent, est sélectionné parmi :

les huiles et les cires hydrocarbonées ; les huiles de silicone ; les esters triglycéridiques ; les esters glycéridiques, les monoglycérides acétylés, les monoglycérides alkoxylés ; les esters alkyls et alkényls d'acides gras ayant 10 à 20 atomes de carbone ; les alcools gras ayant 10 à 20 atomes de carbone ; de la lanoline et les dérivés de celle-ci ; les cires naturelles, les esters de celles-ci et les cires naturelles alkoxylées.

**5.** Composition détergente selon la revendication 2, dans laquelle l'agent d'humectation, si présent, est sélectionné parmi les polyols $C_2$-$C_6$, les glycols de polyéthylène ayant des masses moléculaires d'environ 100 à environ 1 500.

**6.** Composition détergente selon l'une quelconque des revendications précédentes, dans laquelle le matériau émollient de la peau (B) est présent dans une quantité comprise entre 0,1 % et 10 % en poids de la composition.

**7.** Composition détergente selon la revendication 2, dans laquelle :

l'agent d'humidification de la peau (B1), s'il y a, est présent dans une quantité comprise entre 0,02 % à 5 % en poids de la composition ; l'agent d'occlusion (B2), s'il y a, est présent dans une quantité comprise entre 0,1 % et 2 % en poids de la composition; et l'agent d'humectation (B3), s'il y a, est présent dans une quantité allant jusqu'à 15 % en poids de la composition.

**8.** Composition détergente selon la revendication 7, dans laquelle les rapports en poids des trois catégories de matériaux émollients de la peau sont B1:B2:B3 = 0 - 15:2 - 10:5 - 50.

**9.** Composition détergente selon l'une quelconque des revendications précédentes, dans laquelle l'agent détergent actif (A) est présent dans une quantité n'excédant pas 40 % en poids de la composition.

13

**10.** Composition détergent selon l'une quelconque des revendications précédentes dans laquelle le détergent anionique non savonneux (A1) est présent dans une quantité comprise entre 5 % et 20 % en poids de la composition, et le détergent amphotère (A2) est présent dans une quantité comprise entre 1 % et 15 % en poids de la composition.

**11.** Composition détergente selon la revendication 10, dans laquelle le rapport de poids des deux catégories d'agents détergents actifs est A1:A2 = 10:1 à 1:10.

**12.** Composition détergente selon l'une quelconque des revendications précédentes, dans laquelle la proportion en poids de l'agent actif (A) par rapport au matériau émollient de la peau (B) est A:B = 20:1 à 5:1.

**13.** Composition détergente selon la revendication 2, dans laquelle la proportion en poids de l'agent détergent actif moussant (A) par rapport à l'agent d'occlusion (B2) est A:B2 = 10:1 à 100:1.

**14.** Composition détergente selon l'une quelconque des revendications précédentes, dans laquelle le matériau émollient de la peau comprend un agent d'humectation (B3).

**15.** Composition détergente selon l'une quelconque des revendications précédentes, dans laquelle le matériau émollient de la peau comprend un agent d'humectation (B3) réuni avec l'agent d'humidification de la peau (B1).

**16.** Composition détergente selon l'une quelconque des revendications précédentes, dans laquelle le matériau émollient de la peau comprend un agent d'humectation (B3) réuni avec un agent d'occlusion (B2).

**17.** Composition détergente selon l'une quelconque des revendications précédentes, dans laquelle le matériau émollient de la peau comprend :
B1 - 0,02 % à 5 % d'un agent d'humidification de la peau,
B2 - 0,1 % à 2 % d'un agent d'occlusion ; et
B3 - 0 % à 15 % d'un agent d'humectation.

**18.** Composition détergente selon l'une quelconque des revendications précédentes, comprenant en outre une matière supplémentaire ou plus sélectionnée parmi les agents épaississants, les agents nacrants ou les agents opacifiants, les parfums, les colorants, les antioxydants, les agents préservatifs, les acides gras et les savons.

**19.** Composition détergente selon l'une quelconque des revendications précédentes, dans laquelle le détergent anionique actif (A1) comprend de l'iséthionate acyle gras $C_8$ à $C_{22}$.

**20.** Composition détergente sous la forme d'un liquide acqueux de viscosité comprise dans l'intervalle de 10 000 à 1 000 mPas à une vitesse de cisaillement de 10 $s^{-1}$, contenant :
A) 3 % à 40 % d'un détergent composé de
A1) au moins 2 % d'iséthionate acyle $C_8$-$C_{22}$
A2) au moins 1 % d'un détergent amphotère sélectionné parmi les bétaïnes, les sulfobétaïnes et les amidobétaïnes ;
B) 0,1 % à 10 % d'un matériau émollient de peau, dans laquelle la proportion en poids de l'iséthionate acyle (A1) par rapport au détergent (A2) est comprise entre 10:1 et 1:4, et les agents détergents actifs présents dans la composition forment aux mêmes concentrations une solution claire dans de l'eau distillée.

**21.** Composition détergent selon la revendication 20, dans laquelle les agents détergents actifs présents comprennent un détergent anionique supplémentaire.

**22.** Composition détergente selon la revendication 21, dans laquelle la quantité de détergent anionique supplémentaire est telle que l'on conserve la proportion en poids du détergent anionique total par rapport au second détergent (A2) dans l'intervalle de 10:1 à 1:2.

14